# DEMANDE DE BREVET EUROPEEN

(11) **EP 1 386 969 A1**
(43) Date de publication de la demande: **04.02.2004**
(21) Numéro de dépôt: 03291880.7
(22) Date de dépôt: 29.07.2003
(51) Int. Cl.: C12P 7/64

(54) **Procédé de préparation d'acides gras polyinsaturés libres et leurs métabolites d'oxydation**

(30) Priorité: 02.08.2002 FR 0209912
(71) Demandeur: LABORATOIRES GOEMAR S.A., 35400 Saint-Malo (FR)
(72) Inventeur: Bouarab, Kamal, Sherbrook, Québec J1K2R1 (CA); Salaun, Jean-Pierre, 29870 Landeda (FR); Yvin, Jean-Claude, 35400 Saint Malo (FR); Potin, Philippe, 29680 Roscoff (FR)
(74) Mandataire: Koch, Gustave

(57) **Abrégé**

L'invention concerne un procédé de préparation d'acides gras polyinsaturés libres et de leurs métabolites d'oxydation caractérisé par le fait que successivement
- on stimule, dans une algue rouge, la production d'acides gras polyinsaturés et de leurs métabolites d'oxydation par action d'un éliciteur de nature peptidique, lipidique ou saccharidique, puis
- on extrait les acides gras polyinsaturés produits ainsi que leurs métabolites d'oxydation.

## Description

L'invention a pour objet un procédé de préparation d'acides gras polinysaturés libres et de leurs métabolites d'oxydation.

Elle vise plus particulièrement l'application du procédé conforme à l'invention à la préparation des acides
- 12-hydroxy-eicosatetraénoique ou 12-HETE et
- 11,12-epoxy-eicosatriénoique ou 11,12-EET.
qui sont des métabolites d'oxydation de l'acide arachidonique et qui constituent des médicaments importants en ce sens qu'ils restaurent, corrigent ou modifient certaines fonctions organiques de l'être humain ; ces médicaments sont l'objet de la demande de brevet déposée par la Société Demanderesse à la même date que la présente demande sous le titre "Nouveau médicament".

L'invention repose sur le résultat des recherches, menées par la Société Demanderesse et qui lui ont permis de trouver qu'il était possible de stimuler par mise en oeuvre d'éliciteurs de nature protéique, lipidique ou saccharidique la production de ces métabolites dans les algues rouges et plus particulièrement chez *Chondrus Crispus,* dans les réactions de défense desquelles ils jouent un rôle qui n'est pas encore élucidé, sachant qu'il est déjà connu que ces métabolites sont produits à l'issue de la cascade d'oxydation de l'acide arachidonique sous l'action de la lipoxygénase pour le premier et sous l'action d'une enzyme à cytochrome P450 pour le second.

Et le mérite de la Société Demanderesse est d'avoir trouvé que cette stimulation dans *Chondrus Crispus* pouvait avantageusement être obtenue par la mise en oeuvre en tant qu'éliciteurs, de composants produits par l'algue verte *Acrochaete operculata.*

Une fois la stimulation réalisée, résultat qui est obtenu en environ 6 à 12 heures après l'inoculation de l'éliciteur sur le substrat constitué par l'algue rouge, l'acide arachidonique et les métabolites produits sont extraits du tissu végétal de *Chondrus Crispus.*

Il s'ensuit que le procédé conforme à l'invention pour la préparation d'acides gras polyinsaturés libres et de leurs métabolites d'oxydation est caractérisé par le fait que, successivement,
- on stimule, dans une algue rouge, la libération d'acides gras polyinsaturés et la production de leurs métabolites d'oxydation par action d'un éliciteur de nature peptidique, lipidique ou saccharidique, puis
- on extrait les acides gras polyinsaturés libérés ainsi que leurs métabolites d'oxydation.

Selon un mode de réalisation avantageux du procédé conforme à l'invention, la stimulation de la libération des acides gras polyinsaturés et de la production de leurs métabolites d'oxydation est obtenue dans le cas de l'algue rouge *Chondrus Crispus* par élicitation sous l'action de composants de l'algue verte *Acrochaete operculata.*

Selon un autre mode de réalisation avantageux du procédé conforme à l'invention, l'élicitation est réalisée en ayant recours à un extrait *d'Acrochaete operculata,* obtenu par traitement aqueux à froid ou à chaud d'un broyat de l'algue.

Se proposant par conséquent de préparer des acides gras polyinsaturés et leurs métabolites d'oxydation dont notamment le 12-HETE et le 11,12-EET, conformément à l'invention, on procède comme suit ou de façon équivalente.

On cultive tout d'abord des thalles de *Chondrus Crispus.*

Pour ce faire, on procède à la culture de ces thalles [notamment en utilisant une souche haploïde (gamétophytes) identifiée par JC002PC-6 et communiquée par le laboratoire du Professeur Juan Correa de la Faculté des Sciences Biologiques, Université Catholique, Santiago, Chili] dans un milieu de culture désigné par SFC et préparé en ajoutant à 1 litre d'eau de mer, filtrée avec un filtre de 0,2µm, une quantité de 2ml de chacunes des cinq solutions identifiées ci-après et préparées comme indiqué :

### 1. Solution à base de fer.

On dissout 367 mg/l de sels sodium ferrique d'éthylène diamine acide tétra-acétique dans de l'eau distillée.

### 2. Solution à base de phosphates.

On prépare 50mM de (NaH₂PO₄, H₂O) dans de l'eau distillée.

### 3. Solution à base de nitrates.

On prépare 1M de NaNO₃ dans de l'eau distillée.

### 4. Solution à base de métaux.

On prépare séparément des solutions de 14mg/ml de (MnCl₂, 4H₂O), de 1mg/ml de ZnCl₂, de 47/µg/ml de (CoCl₂, 6H₂O) et de 0.04µg/ml de (CuCl₂, 2H₂O) dans de l'eau distillée; un volume de 50ml de chacune de ces solutions est ajouté à une solution de Na₂EDTA (4.36g/l); on fait bouillir l'ensemble pendant 10 minutes, on ajoute 1 litre d'eau distillée et on ajuste le pH à 7.5 avant de filtrer avec un filtre de 0.2 µm.

### 5. Solution à base de vitamines.

On prépare séparément des solutions de biotine (0.5mg/l), d'acide folique (1mg/l), de thiamine B1 (1.5 mg/l) et de B12 (0.5mg/l) dans de l'eau distillée; on ajoute 1.25ml de chacune de ces solutions à 250 ml d'eau de mer filtrée avec un filtre de 0.2 µm.

On cultive, par ailleurs, en utilisant de nouveau le milieu défini ci-dessus, des isolats *d'Acrochaete operculata* (notamment des isolats obtenus du Laboratoire du Professeur Juan Correa et qui sont constitués par des cultures unialgales établies à partir de thalles de *Chondrus Crispus* infectés par cet endophyte dans la nature, récoltés au Canada en 1987 et identifiés par KH 040687-1-1.

Les deux cultures sont maintenues à 15°C avec une photopériode de 16/8 (jour/nuit) et un flux lumineux de 40 µmol m-2 s-1.

Le milieu de chaque culture est renouvelé chaque semaine.

Après 4 mois de culture, lorsque la biomasse *d'Acrochaete operculata* atteint une quantité d'environ 5g d'algues fraîches égouttées pour 5 litres de milieu de culture, une fraction de 1.5g d'algues est prélevée, filtrée sur du papier Whatman 3MM et égouttée par pressage.

Cette biomasse est alors congelée dans l'azote liquide et stockée au congélateur à -80°C.

Pour préparer les extraits acellulaires *d'Acrochaete operculata,* on utilise la susdite fraction de 1.5g d'*Acrochaete operculata* congelée dans l'azote liquide, on la réduit en poudre dans l'azote liquide, et, après évaporation de l'azote, la poudre est reprise dans 1ml de tampon d'extraction constitué de 0.5M Tris HCl pH 6.5, de 50mM de NaCl et 10mM de MgCl₂.

L'extraction peut se faire à froid ou à chaud à 100°C pendant 15minutes.

Après centrifugation à 12000g pendant 30 minutes, le surnageant est récupéré.

Ce surnageant constitue l'extrait utilisé en tant qu'éliciteur.

La culture de *Chondrus Crispus,* dont il a été question ci-dessus, est poursuivie pendant 4 à 8 mois.

A la fin de cette culture, on prélève un échantillon de 500mg (poids frais) de gamétophytes.

Cet échantillon est utilisé pour l'élicitation.

Pour cette élicitation, on incube pendant 1 heure les 500mg de gamétophytes dans 10ml d'eau de mer, filtrée avec un filtre 0,2µm, en présence d'une quantité de 70µl du susdit extrait *d'Acrochaete operculata.*

On induit ainsi l'activation des phospholipases qui libèrent des acides gras polyinsaturés libres, dont l'acide arachidonique, ainsi que les lipoxygénases et le cytochrome P450 sous l'action respective desquels sont formés, à partir de l'acide arachidonique, les métabolites 12-HETE et 11,12-EET.

Une fois l'incubation terminée, (la fin de l'incubation peut être contrôlée par congélation des algues dans l'azote liquide), on récupère par extraction les acides gras et les métabolites formés.

Pour ce faire, on peut procéder successivement à une extraction aqueuse de l'algue rouge broyée dans l'azote liquide suivie d'une extraction par solvant organique.

On prépare tout d'abord un extrait aqueux des gamétophytes qui ont été soumis à l'élicitation par l'extrait *d'Acrochaete operculata.*

Pour ce faire, on soumet une quantité de 5g de la culture de gamétophytes incubée avec les extraits de l'algue verte *Acrochaete operculata* à un broyage à l'azote liquide.

Le produit résultant de ce broyage est mis en suspension dans 20ml d'un tampon Tris-HCI 50mM à pH 9.5 contenant 500 mM de KCl et 10 mM de β-mercaptoéthanol.

Après homogénéisation par agitation lente dans de la glace, l'extrait est centrifugé à 12 000g pendant 10 minutes, puis les protéines contenues dans le surnageant sont dosées par la méthode de Bradford (1976). Le réactif de dosage utilisé est un produit commercial à base d'un colorant, le bleu de Coomassie et vendu sous la marque Bio-Rad protein assay; il s'agit d'une forme anionique du colorant qui se fixe préférentiellement aux protéines par interaction avec leurs groupements cationiques.

Les échantillons sont tout d'abord solubilisés dans un tampon MgCl₂ 10mM, NaCl 50 mM, perfablock 1mM, Tris HCl 50 mM à pH 7.5 et centrifugés pendant 10 minutes à 12000g.

A 800µl d'extrait dilué, contenant entre 1 et 10 µg de protéines, on ajoute 200 µl du susdit réactif Bio-Rad et on mesure la densité optique à 595 nm à l'aide d'un spectrophotomètre.

L'étalonnage est réalisé à l'aide d'une gamme étalon effectuée avec de la albumine sérique de boeuf.

On détermine ainsi un rendement d'extraction des protéines solubles.

C'est à partir du susdit extrait aqueux que l'on récupère les acides gras et leur métabolites.

Cet extrait qui contient 24mg de protéines est dilué, pour obtenir un volume de 40ml, avec du tampon 100mM Tris pH 8.5.

Les métabolites sont extraits 2 fois avec 60mL de diéthylether.

La phase organique est évaporée à sec sous flux d'azote et les résidus sont redissous dans de l'éthanol absolu.

Une partie aliquote est évaporée et redissoute dans 100 µl d'acétonitrile.

Une quantité de 40 µl est injectée pour analyse en RP-HPLC couplée à un détecteur APCI-ESI-MS pour identification à partir de la comparaison du spectre de masse du métabolite avec celui d'un standard authentique et quantification par étalonnage avec des quantités connues des métabolites d'acides gras comme le 12-HETE et le 11,12-EET.

La préparation finale contient 1.5 µg de 12-HETE et une quantité inférieure ou égale à 0.5 µg de 11,12-EET. Le rendement final de la préparation est de 0,3 mg de 12-HETE et de 0,1mg de 11,12 EET par kilogramme d'algue fraîche.

Ces métabolites sont stables dans l'éthanol pendant plusieurs mois et sont conservés à -20°C.

D'autres métabolites dont des hydroperoxides (12-HPETE, 13 HPOTE, 13-HPODE ou des céto alcools ou des époxy-, et des dérivés hydroxylés des acides arachidonique, linolénique ou linoléïque sont également identifiés et, compte tenu de leurs activités biologiques sur des systèmes animaux ou végétaux, ces composés obtenus par la mise en oeuvre du procédé, objet de la présente invention, pourraient donner lieu à de nouvelles applications.

## Revendications

1. Procédé de préparation d'acides gras polyinsaturés libres et de leurs métabolites d'oxydation **caractérisé par le fait que** successivement
- on stimule, dans une algue rouge, la production d'acides gras polyinsaturés et de leurs métabolites d'oxydation par action d'un éliciteur de nature peptidique, lipidique ou saccharidique, puis
- on extrait les acides gras polyinsaturés produits ainsi que leurs métabolites d'oxydation.

2. Procédé selon la revendication 1, **caractérisé par le fait que** la stimulation de la production des acides gras polyinsaturés et de leurs métabolites d'oxydation est obtenue dans le cas de l'algue rouge *Chondrus Crispus* par élicitation sous l'action de composants de l'algue verte *Acrochaete operculata.*

3. Procédé selon la revendication 2, **caractérisé par le fait que** l'élicitation est réalisée en ayant recours à un extrait *d'Acrochaete operculata,* obtenu par extraction aqueuse à froid ou à chaud de l'algue verte broyée dans l'azote liquide.

4. Procédé selon l'une des revendications 1 à 3 **caractérisé par le fait que** l'extraction des acides gras polyinsaturés et de leurs métabolites d'oxydation est réalisée par extraction aqueuse de l'algue broyée dans l'azote liquide, suivie d'une extraction par solvant organique.
